# EUROPEAN PATENT SPECIFICATION

(11) **EP 3 017 779 B1**
(45) Date of publication and mention of the grant of the patent: **31.05.2017**
(21) Application number: 15201674.7
(22) Date of filing: 07.03.2011
(51) Int. Cl.: A61B 17/56, A61C 5/00, A61F 2/28, A61B 17/88

(54) **BONE CEMENT INJECTION PUNCTURE NEEDLE**
PUNKTURNADEL ZUR KNOCHENZEMENTINJEKTION
AIGUILLE DE PONCTION D'INJECTION DE CIMENT OSSEUX

(30) Priority: 09.03.2010 JP 2010052300
(43) Date of publication of application: 11.05.2016
(62) Divisional of application: 11753311.7
(73) Proprietor: St. Marianna University School of Medicine, Kawasaki-shi Kanagawa 216-8511 (JP); Terumo Kabushiki Kaisha, Tokyo 151-0072 (JP)
(72) Inventor: TAKIZAWA, Kenji, Kawasaki-shi,, Kanagawa 216-8511 (JP); HAYAKAWA, Koichi, Kanagawa 259-0151, Japan (JP)
(74) Representative: TBK

(56) References cited:
- WO-A1-2008/114483
- US-A- 5 800 439
- US-A1- 2004 097 880
- US-A1- 2007 197 935
- US-A1- 2008 119 821

## Description

### Technical Field

The present invention relates to a puncture needle for injecting bone cement into a bone.

### Background Art

Percutaneous vertebroplasty is a therapeutic method which is used to alleviate pain caused by a compression fracture of a vertebral body of the patient by injecting bone cement into the injured area of the vertebral body to reinforce the vertebral body. Percutaneous vertebroplasty is a relatively new treatment technique that was first performed in France in 1987, and is now conducted in many medical facilities throughout Japan.

Basically, percutaneous vertebroplasty is based on a transpedicular approach, wherein a hollow puncture needle is inserted into a vertebral body through the pedicle that lies horizontally on the back of the vertebral body, and bone cement is injected into the vertebral body through a passage in the hollow puncture. Generally, a bone biopsy needle is used as the puncture needle for injecting bone cement. For details, see Japanese Laid-Open Patent Publication No. 2003-024339, for example. The transpedicular approach includes a two-needle method in which two needles are inserted respectively into left and right sides of a vertebral body, and a single-needle method in which a single needle is inserted into one of left and right sides of a vertebral body. The single-needle method is considered to be preferable because it is less costly and less liable to cause complications, requires a smaller radiation dosage, and can be performed in a shorter time than the two-needle method.

US 2008/0119821 A1 discloses a needle comprising the features of the preamble of claim 1.

US 2004/0097880 A1 discloses a needle structure comprising an outer needle of hollow constitution, and an inner needle. The outer needle has side hole slits on the side of a shaft portion of the outer needle to permit fluid to exit in response to a pressure level created by introduction of fluid into the lumen by a syringe.

WO 2008/114483 A1 discloses a unit for resetting bone fracture in which cement leakage into the extrapyramidal tract is to be prevented. The unit comprises an inlet tube whereby the front end is introduced into a space in a pyramid via a through hole formed in the pedicle of vertebral arch, a balloon made of an elastic filmy material which is detachably attached to the front end of the above-described inlet tube and can be inserted in the deflated state into the pyramid via the through hole and inflated in the pyramid, and a cement which is supplied into the balloon through the inlet tube, wherein the inlet tube is provided with an air vent part for discharging air enclosed in the inlet tube and the balloon.

US 5 800 439 A also discloses a needle comprising the features of the preamble of claim 1.

US 2007/0197935 A1 discloses a needle structure comprising a cannula as an outer needle of hollow constitution, and a trocar as an inner needle. The trocar has a handle. The cannula is set on the trocar.

### Summary of Invention

However, puncture needles that have heretofore been used are disadvantageous in that when bone cement is injected by the single-needle method, the bone cement may possibly leak out from the bone.

More specifically, when bone cement is injected by the single-needle method using a conventional puncture needle, the internal pressure in the bone increases as the bone cement is injected, which causes the bone cement to leak out from the bone (e.g., into a lumen of the vertebral canal or a vein). Consequently, it has been recommended to perform the two-needle method, so as to allow the internal pressure of the bone to be reduced using one of the needles, while placing more emphasis on avoiding the problem of internal pressure buildup than on the advantages of the single-needle method, which is preferable for both the patient and the surgeon.

The present invention has been made in view of the foregoing problems. It is an object of the present invention to provide a bone cement injection puncture needle, which is capable of injecting bone cement into a bone without increasing the internal pressure of the bone according to the single-needle method.

The object is solved by a bone cement injection puncture needle having the features of claim 1.

Further developments are stated in the dependent claims.

With the above arrangement, simple constitution is achieved which enables fluid communication to be established between the interior of the bone and the proximal end side hole. More specifically, since the depressurization passage, which opens at the most distal end portion of the outer needle, is defined between the outer needle and the inner tube, when bone cement is injected into the bone while the outer needle punctures the bone and the inner tube is inserted in the outer needle, gas or liquid in the bone is made to flow from the foremost end opening of the outer needle into the depressurization passage between the outer needle and the inner tube. Such gas or liquid then flows through the depressurization passage and flows out of the proximal end side hole.

When the inner tube is inserted in the outer needle, a distal end portion of the inner tube may project from a distal end portion of the outer needle.

With the above arrangement, a step between the distal end portion of the inner tube and the distal end portion of the outer needle serves as a marker that is used when carrying out image guidance (X-ray fluoroscopy or CT fluoroscopy). Since the step can visually be recognized easily in the image, the outer needle can be inserted simply and reliably into the bone. Since bone cement, which has flowed out from the distal end of the inner tube does not become adhered to the interior of the outer needle, the inner needle can reliably be inserted again into the outer needle after the inner tube has been pulled out. Since bone cement is not adhered to the interior of the outer needle, bone cement is not pushed into the bone when the inner needle is inserted again into the outer needle. Further, since more bone cement than necessary is prevented from being injected into the bone, an accurate amount of bone cement can be injected into the bone.

### Brief Description of Drawings

FIG. 1 is an overall view of a bone cement injection puncture needle according to a first unclaimed example;
FIG. 2 is a cross-sectional view, partially omitted from illustration, taken along line II-II of FIG. 1;
FIG. 3 is a cross-sectional view, partially omitted from illustration, of the bone cement injection puncture needle according to the first embodiment, with an inner tube inserted in an outer needle;
FIG. 4 is an enlarged view, partially omitted from illustration, showing a distal end portion and a nearby portion of the outer needle of the bone cement injection puncture needle according to the first embodiment;
FIG. 5A is a view showing the manner in which the outer needle and an inner needle are inserted into a bone;
FIG. 5B is a view showing the manner in which the inner needle is removed from the outer needle;
FIG. 5C is a view showing the manner in which the inner tube is inserted in the outer needle;
FIG. 5D is a view showing the manner in which a syringe filled with bone cement is connected to an inner tube hub;
FIG. 6A is a view showing the manner in which bone cement is injected into the bone, whereupon a gas or liquid in the bone flows out of the body through a depressurization passage;
FIG. 6B is a view showing the manner in which the inner tube is removed from the outer needle;
FIG. 6C is a view showing the manner in which the inner needle is mounted again in the outer needle and then is removed from the bone;
FIG. 7 is a cross-sectional view, partially omitted from illustration, of a bone cement injection puncture needle according to a first embodiment of the present invention;
FIG. 8 is a cross-sectional view, partially omitted from illustration, of the bone cement injection puncture needle according to the first embodiment, with an inner tube inserted in an outer needle;
FIG. 9A is a view showing the manner in which the outer needle and an inner needle are inserted into a bone;
FIG. 9B is a view showing the manner in which the inner needle is removed from the outer needle;
FIG. 9C is a view showing the manner in which the inner tube is inserted in the outer needle;
FIG. 9D is a view showing the manner in which a syringe filled with bone cement is connected to an inner tube hub;
FIG. 10A is a view showing the manner in which bone cement is injected into the bone, whereupon gas or liquid in the bone flows out of the body through a depressurization passage;
FIG. 10B is a view showing the manner in which the inner tube is removed from the outer needle;
FIG. 10C is a view showing the manner in which the inner needle is mounted again in the outer needle and then is removed from the bone;
FIG. 11 is a side elevational view of an outer needle hub and other components according to a second unclaimed example; and
FIG. 12 is a cross-sectional view, partially omitted from illustration, of a bone cement injection puncture needle according to a third unclaimed example.

### Description of Embodiments

Bone cement injection puncture needles according to preferred embodiments of the present invention will be described below with reference to the accompanying drawings. In the present description, the term "bone cement" refers not only to bone cement (such as a plastic product) but also to bone paste (such as a calcium phosphate product). [First unclaimed example]

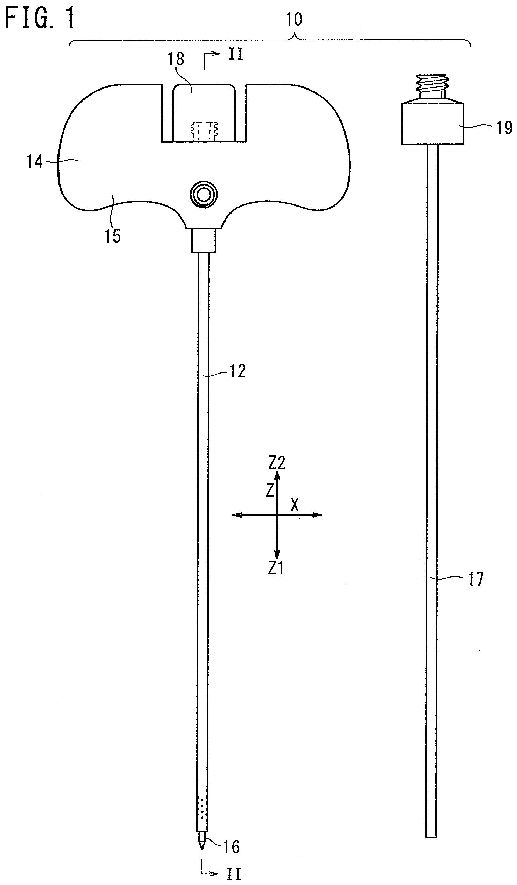

FIG. 1 is an overall view of a bone cement injection puncture needle 10 (hereinafter referred to as a "puncture needle 10") according to a first unclaimed example which does not form part of the invention but is useful for understanding the invention. As shown in FIG. 1, the puncture needle 10 comprises an outer needle 12 having hollow constitution, an outer needle hub 14 fixed to a proximal end portion of the outer needle 12, an inner needle 16 which is insertable in the lumen in the outer needle 12, an inner needle hub 18 fixed to a proximal end portion of the inner needle 16, an inner tube 17 which is insertable in the lumen in the outer needle 12, and an inner tube hub 19 fixed to a proximal end portion of the inner tube 17. In FIG. 1, the inner needle 16 is shown as being inserted into the outer needle 12, while the inner tube 17 is shown as being removed from the outer needle 12.

In the following description, axial directions of the inner needle 16 and the outer needle 12 are referred to as Z directions, directions perpendicular to the Z directions are referred to as X directions, and directions perpendicular to the Z directions and the X directions are referred to as Y directions. In FIG. 1, the X directions are perpendicular to the Z directions and the X and Z directions are parallel to the sheet of the drawing, whereas the Y directions are perpendicular to the sheet of the drawing. Among the Z directions, the direction toward the distal end portion of the puncture needle 10 is represented by Z1, and the direction toward the proximal end portion of the puncture needle 10 is represented by Z2.

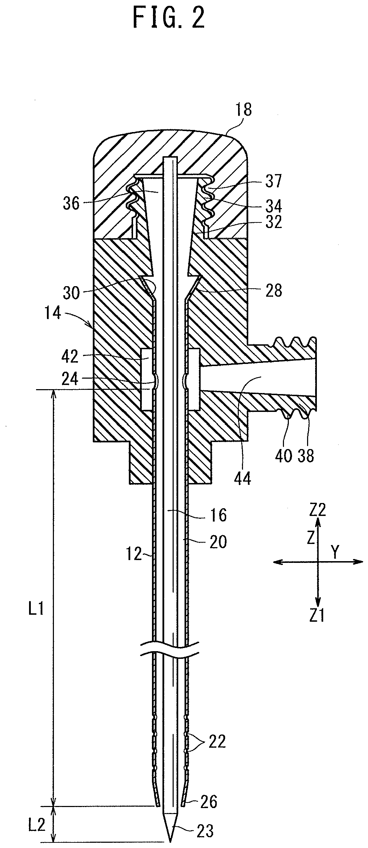

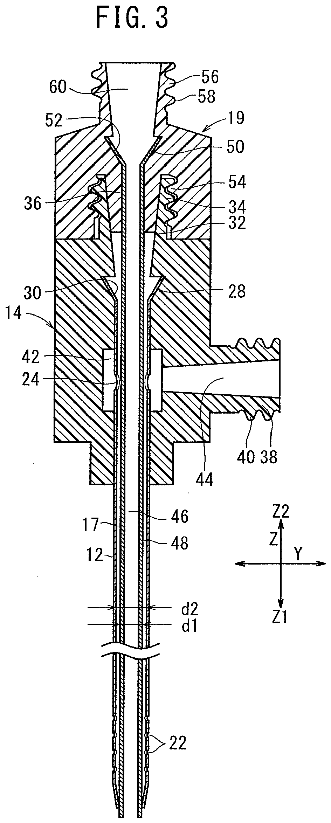

FIG. 2 is a cross-sectional view, partially omitted from illustration, taken along line II-II of FIG. 1. As shown in FIG. 2, the outer needle 12 comprises a hollow member with opposite open ends. The outer needle 12 may be in the form of a hollow cylindrical tube. The inner needle 16 can be inserted into a lumen 20 in the outer needle 12. The length of the outer needle 12 lies within a range from about 90 to 200 mm, and the inside diameter d2 (see FIG. 3) of the outer needle 12 lies within a range from about 1.5 to 3.3 mm, for example.

The outer needle 12 may be made of any materials as long as such materials are strong enough not to become damaged or deformed when the outer needle 12 is inserted into and pulled out from a bone. Preferably, the outer needle 12 is made from a metallic material such as stainless steel, aluminum alloy, copper alloy, or the like, for example.

The outer needle 12 has first side holes (distal-end holes) 22 formed in a side wall near the distal end portion thereof. The first side holes 22 extend between inner and outer spaces of the outer needle 12, and preferably are provided as a plurality of holes distributed in circumferential and axial directions of the outer needle 12. The number of first side holes 22 is preferably in a range from 4 to 36, and more preferably, in a range from 10 to 26. The preferred layout and dimensions of the first side holes 22 will be described later.

The outer needle 12 has second side holes (proximal-end holes) 24 formed therein near the proximal end portion thereof. The second side holes 24 extend between inner and outer spaces of the outer needle 12. The second side holes 24, i.e., regions thereof closest to the distal end (in the Z1 direction), are spaced from the foremost end of the outer needle 12 by a distance L1, which is set such that when the outer needle 12 with the inner needle 16 inserted therein is inserted into a bone, the second side holes 24 are reliably positioned outside the body of the patient. More specifically, the distance L1 is equal to or greater than 80 mm, and more preferably, is equal to or greater than 120 mm.

Although the outer needle 12 may have only a single second side hole 24, preferably the outer needle 12 includes a plurality of second side holes 24 distributed in circumferential and axial directions. As shown in FIG. 2, two second side holes 24 are spaced from each other in the circumferential direction. The first side holes 22 and the second side holes 24 are held in fluid communication with each other through the lumen 20 of the outer needle 12.

The outer needle 12 includes a tapered portion 26 on a distal end portion thereof, the tapered portion 26 being progressively tapered off toward the distal end. The angle of the tapered portion 26 with respect to the axis of the outer needle 12 is set to a value within a range from about 1 to 30 degrees, for example. The distal end portion of the inner needle 16 is supported by an inner circumferential surface of the tapered portion 26.

The outer needle 12 includes a flaring portion 28 on a rear end portion thereof. The flaring portion 28 spreads conically toward a proximal end (in the Z2 direction). The angle of the flaring portion 28 with respect to the axis of the outer needle 12 is set to a value within a range from about 15 to 60 degrees, for example. The flaring portion 28 has an outer circumferential surface in abutment with and supported by a tapered support 30 formed on the outer needle hub 14.

The outer needle hub 14 comprises a member that is coupled to the proximal end portion of the outer needle 12, and has an integral grip 15 (see FIG. 1) extending in directions perpendicular to the axis of the outer needle 12 (in the X directions as illustrated). As shown in FIG. 2, the outer needle hub 14 is insert-molded in a manner so as to cover and be fixed to the proximal end portion of the outer needle hub 14.

The outer needle hub 14 is not limited to any particular materials, but may be made of polyester such as polyvinyl chloride, polyethylene, polypropylene, cyclic polyolefin, polystyrene, poly(4-methylpentene-1), polycarbonate, acrylic resin, acrylonitrile-butadiene-styrene copolymer, polyethylene terephthalate, polyethylene naphthalate, or the like, butadiene-styrene copolymer, polyamide (e.g., nylon 6, nylon 6·6, nylon 6·10, nylon 12), or the like.

The outer needle hub 14 includes a main connection port (first port) 32 formed in an upper end portion thereof (in the Z2 direction) and which is held in fluid communication with the lumen 20 of the outer needle 12 through the proximal end opening of the outer needle 12. The main connection port 32 has an externally threaded outer circumferential surface 34 for screwed connection with the inner needle hub 18, and also for screwed connection with the inner tube hub 19. The outer needle hub 14 also has a first passage 36 formed therein, which extends from the open mouth of the main connection port 32 to a position that confronts the open end of the outer needle 12.

The outer needle hub 14 also includes an auxiliary connection port (third port) 38 on one side surface thereof (a surface facing in a Y direction). The auxiliary connection port 38 is held in fluid communication with the lumen 20 of the outer needle 12 through the second side holes 24. The auxiliary connection port 38 has an externally threaded outer circumferential surface 40 for connection to another device or constitution. The outer needle hub 14 has a second passage 42 formed therein, which surrounds the outer needle 12 and is held in fluid communication with the second side holes 24, and a third passage 44 formed therein, which extends from the second passage 42 to the opening of the auxiliary connection port 38.

If one or more second side holes 24 are formed in a region that faces toward the auxiliary connection port 38, then the outer needle hub 14 may have a flow channel formed therein, in which the second passage 42 is dispensed with, and the third passage 44 extends to a position that confronts the second side holes 24.

The inner needle 16 comprises a bar-shaped member, which is inserted into the lumen 20 of the outer needle 12 and has a sharp needle point 23 on the distal end thereof. The inner needle 16 may be made of any materials, such as stainless steel, aluminum alloy, copper alloy, or the like, for example, insofar as such materials are strong enough not to become damaged or deformed when the inner needle 16 is inserted into a bone.

The inner needle 16 has a length set to a value such that when the inner needle hub 18 is connected to the outer needle hub 14, the distal end of the inner needle 16 projects slightly from the distal end of the outer needle 12. With the inner needle hub 18 connected to the outer needle hub 14, the length by which the inner needle 16 projects from the distal end of the outer needle 12 (i.e., the distance L2 between the distal end of the inner needle 16 and the distal end of the outer needle 12) preferably is set to a value within a range from 2 to 10 mm. The needle point 23 should be fully protruded from the distal end of the outer needle 12 when the inner needle hub 18 is connected to the outer needle hub 14.

The inner needle 16 has an outside diameter, which is substantially the same as the inside diameter of the most distal end portion of the outer needle 12. More specifically, the outside diameter of the inner needle 16 may be set to a value that allows the inner needle 16 to be smoothly inserted into the lumen 20 of the outer needle 12, with essentially no gap created between the outer circumferential surface of the inner needle 16 and the inner circumferential surface of the most distal end portion of the outer needle 12.

The inner needle hub 18 comprises a member that is coupled to the proximal end portion of the inner needle 16. The inner needle hub 18 is constructed such that the inner needle hub 18 can be mounted on and removed from the outer needle hub 14 without the need for special tools. The inner needle hub 18 has an internally threaded surface 37, which can be screwed on the externally threaded outer circumferential surface 34 of the main connection port 32 of the outer needle hub 14. The inner needle hub 18 becomes fixed to the outer needle hub 14 when the inner needle hub 18 is screwed on the main connection port 32.

The outside diameter of the inner needle hub 18 is greater than the outside diameter of the inner needle 16. More specifically, the outside diameter of the inner needle hub 18 is set to a value which allows the user (a medical worker such as a doctor or the like) to hold and to push, pull or turn the inner needle hub 18 with ease. The inner needle hub 18 is not limited to any particular materials, but may be made of the same material as the outer needle hub 14, e.g., a hard resin such as polycarbonate or the like.

FIG. 3 is a cross-sectional view, partially omitted from illustration, showing the manner in which the inner tube 17 is inserted into the outer needle 12. As shown in FIG. 3, the inner tube 17 has opposite open ends and also has a bone cement passage 46 formed therein. The inner tube 17 has a length in a range from about 100 to 210 mm, which may be set to a value such that when the inner tube hub 19 is mounted on the outer needle hub 14, the most distal end portion of the inner tube 17 is aligned, i.e., lies flush with, the most distal end portion of the outer needle 12, or projects slightly from the outer needle 12.

In FIG. 3, the inner tube 17 comprises a hollow cylindrical tube having an inside diameter ranging from 1.8 to 2.1 mm. The inner tube 17 has an outside diameter d1, which is smaller than the inside diameter d2 of the outer tube. When the inner tube 17 is inserted in the outer needle 12, a depressurization passage 48 is formed between the outer needle 12 and the inner tube 17, which provides fluid communication between the first side holes 22 and the second side holes 24. The outside diameter d1 of the inner tube 17 may be substantially the same as the inside diameter of the most distal end portion of the outer needle 12.

The inner tube 17 includes a flaring portion 50 on a proximal end portion thereof. The flaring portion 50 spreads conically toward the proximal end (in the Z2 direction). The angle of the flaring portion 50 with respect to the axis of the inner needle 16 is set to a value within a range from about 15 to 60 degrees, for example. The flaring portion 50 has an outer circumferential surface, which is in abutment with and supported by a tapered support 52 of the inner tube hub 19.

The inner tube hub 19 is a member that is coupled to the proximal end portion of the inner tube 17, and is constructed such that the inner tube hub 19 can be mounted on and removed from the outer needle hub 14. The inner tube hub 19 has an internally threaded surface 54, which can be screwed on the externally threaded outer circumferential surface 34 of the main connection port 32 of the outer needle hub 14. The inner tube hub 19 is fixed to the outer needle hub 14 when the inner tube hub 19 is screwed on the main connection port 32.

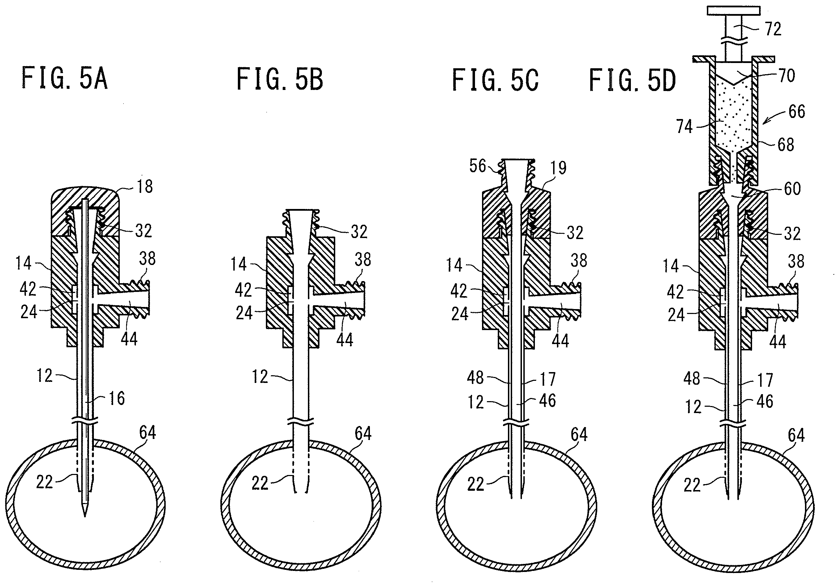

The inner tube hub 19 has an injection port (second port) 56 formed in an upper end portion thereof (in the Z2 direction), which is held in fluid communication with the bone cement passage 46 through the proximal end opening of the inner tube 17. The injection port 56 serves to supply (deliver) bone cement to the inner tube 17. The injection port 56 has an externally threaded outer circumferential surface 58 for screwed connection to a syringe 66 (see FIG. 5D), which serves as an injection device. The inner tube hub 19 also has a lumen 60 formed therein that extends from the opening of the injection port 56 to a position confronting the proximal end opening of the inner tube 17.

The outside diameter of the inner tube hub 19 is greater than the outside diameter of the inner tube 17. More specifically, the outside diameter of the inner tube hub 19 is set to a value that allows the user to hold and to push, pull or turn the inner tube hub 19 with ease. The outside diameter of the inner tube hub 19 may be substantially the same as the outside diameter of the inner needle hub 18. The inner tube hub 19 is not limited to any particular materials, but may be made of the same material as the outer needle hub 14, e.g., a hard resin such as polycarbonate or the like.

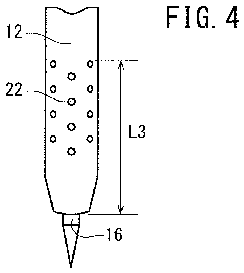

FIG. 4 is an enlarged view, partially omitted from illustration, showing the first side holes 22 and a nearby portion of the outer needle 12. The distance L3 from the foremost end of the outer needle 12 to the first side holes 22 that are positioned closest to the proximal end of the outer needle 12, i.e., a region of the first side holes 22 that is closest to the proximal end of the outer needle 12, is set to a value such that when the outer needle 12 is inserted into a bone, the first side holes 22, which are positioned closest to the proximal end of the outer needle 12, are not positioned outside of the bone, or stated otherwise, such that all the first side holes 22 are positioned within the bone. More specifically, the distance L3 is equal to or smaller than 20 mm, and more preferably, is equal to or smaller than 15 mm.

If the outer needle 12 has a number of first side holes 22, then the first side holes 22 may be positioned in a zigzag pattern (staggered pattern) circumferentially about the outer needle 12. For example, the first side holes 22 may be grouped into rows made up of first side holes 22 that extend along the axis of the outer needle 12, and first side holes 22 of adjacent rows, which are axially displaced with respect to each other. If arranged in this manner, the first side holes 22 are positioned in a well balanced fashion on the outer needle 12, so that the region of the outer needle 12 in which the first side holes 22 are located is prevented from being reduced in mechanical strength.

The first side holes 22 need not be of the same size, but may have different sizes. For example, the diameters of the first side holes 22 may become progressively greater toward the distal end of the outer needle 12, so that when a cleaning device is connected to the auxiliary connection port 38 in order to clean the interior of the bone with a cleaning liquid, the amount of cleaning liquid ejected from the first side holes 22 closest to the proximal end, i.e., closest to the auxiliary connection port 38, will not be greater than the amount of cleaning liquid ejected from the first side holes 22 closest to the distal end. The first side holes 22 need not be circular in shape, as shown in FIG. 4, but may be elliptical or polygonal in shape, or may have different mixed shapes.

The first side holes 22 may be set to a size that allows gas and liquid (e.g., exudate and blood) in the bone to flow smoothly into the outer needle 12. If the first side holes 22 are circular in shape, then the diameter of the first side holes 22 preferably is in a range from 0.3 to 0.7 mm. If the first side holes 22 are of a non-circular shape, then the dimension of the narrowest region thereof should be in a range from 0.3 to 0.7 mm.

If the first side holes 22 are too small, then liquid from within the bone tends to stick and remain within the first side holes 22. However, since the size of the first side holes 22 is set at the above lower limitation, liquid from within the bone is less liable to become stuck within the first side holes 22. On the other hand, if the first side holes 22 are too large, then the outer needle 12 suffers greater resistance upon insertion thereof into a bone, making it less smooth for the user to manually operate the puncture needle. However, since the size of the first side holes 22 is set at the above upper limitation, any increase in resistance suffered by the outer needle 12 upon insertion thereof into the bone is reduced.

The puncture needle 10 according to the first unclaimed example is basically constituted as described above. Operations and advantages of the puncture needle 10 will be described below.

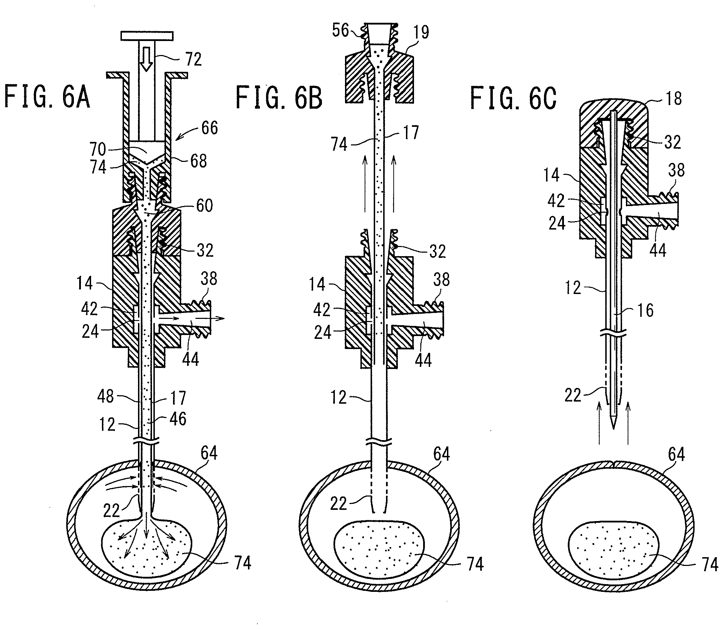

FIGS. 5A to 5D and FIGS. 6A to 6C are views illustrative of a process of injecting bone cement into a bone using the puncture needle 10. For injecting bone cement into a bone using the puncture needle 10, a puncture position and a puncture target are determined while carrying out image guidance (X-ray fluoroscopy or CT fluoroscopy). Thereafter, an assembly including the outer needle 12 and the outer needle hub 14, which are mounted respectively on the inner needle 16 and the inner needle hub 18, is hit by a hammer until the assembly is inserted into the bone 64, which serves as a puncture target (see FIG. 5A). At this time, the assembly is inserted until all of the first side holes 22 become positioned within the bone 64. When the outer needle 12 and the inner needle 16 are inserted into the bone, the second side holes 24 remain positioned outside of the body. The bone 64, which serves as the puncture target, may be a vertebra, for example.

Before the outer needle 12 is inserted into the bone, a tube for supplying a cleaning liquid may be connected to the auxiliary connection port 38, and cleaning liquid may be supplied through the second side holes 24 into the outer needle 12 in order to clean the interior of the outer needle 12.

After the puncture needle 10 has been inserted in the bone 64, the inner needle 16 is removed from the outer needle 12 while leaving the outer needle 12 inserted in the bone 64 (see FIG. 5B). Then, the inner tube 17 is inserted into the outer needle 12, and the inner tube hub 19 is connected to the main connection port 32 of the outer needle hub 14 (see FIG. 5C). As a result, a depressurization passage 48 is formed between the outer needle 12 and the inner tube 17, which provides fluid communication between the first side holes 22 and the second side holes 24 while the outer needle 12 remains inserted in the bone 64.

Before the inner tube 17 is inserted into the outer needle 12, a tube for supplying a cleaning liquid may be connected to the main connection port 32, and cleaning liquid may be supplied through the second passage 42 into the bone cement passage 46 in the inner tube 17 in order to clean the bone cement passage 46.

After the inner tube 17 has been inserted into the outer needle 12, a tube for supplying a cleaning liquid or a syringe filled with bone cement may be connected to the auxiliary connection port 38. Then, cleaning liquid may be supplied through the second passage 42 into the depressurization passage 48 formed between the outer needle 12 and the inner tube 17 in order to clean the depressurization passage 48.

Then, a syringe 66, which serves as an injection device and is filled with bone cement 74, is connected to the injection port 56 (seed FIG. 5D). The syringe 66 has an outer tube 68 the distal end portion of which is connectable to the injection port 56 by screwing, and a pusher 72 having a gasket 70 on a distal end thereof, which is slidably movable in the outer tube 68. The outer tube 68 is filled with bone cement 74.

Then, the bone cement 74 in the syringe 66 is injected through the lumen 60 of the inner tube hub 19 and the bone cement passage 46 into the bone 64 (see FIG. 6A). At this time, gas or liquid in the bone 64 in an amount corresponding to the injected bone cement 74 flows from the first side holes 22 into the depressurization passage 48, and then flows in the depressurization passage 48 and out of the body through the second side holes 24, the second passage 42, and the third passage 44. Therefore, pressure buildup is prevented from developing in the bone 64 upon injection of bone cement 74 into the bone 64, whereby the bone cement 74 is prevented from leaking out from the bone 64.

A suction device, e.g., a syringe or the like, may be connected to the auxiliary connection port 38 to assist in discharging gas or liquid from the bone, while at the same time bone cement 74 is injected into the bone 64. Alternatively, while the inner tube 17 is inserted in the outer needle 12 that has punctured the bone 64, a suction device may be connected to the auxiliary connection port 38, and gas or liquid in the bone 64 may be drawn out before bone cement 74 is injected into the bone 64, thereby developing a negative pressure in the bone 64. After developing such a negative pressure in the bone 64, bone cement 74 may be injected into the bone 64. In this manner, pressure in the bone 64 can be prevented from increasing upon injection of bone cement 74 into the bone 64.

After a predetermined amount of bone cement 74 has been injected into the bone 64, the inner tube 17 is pulled out from the outer needle 12 while the outer needle 12 remains inserted in the bone 64. At this time, bone cement 74 does not become adhered to the interior of the outer needle 12 since the bone cement 74 is removed from within the outer needle 12 at the same time that the inner tube 17 is pulled out.

Then, the inner needle 16 is inserted again into the outer needle 12, whereupon the inner needle hub 18 is connected to the outer needle hub 14. At this time, as described above, no bone cement 74 remains in the outer needle 12. Consequently, the inner needle 16 can reliably be inserted again into the outer needle 12. When the inner needle 16 is reinserted, bone cement 74 is not pushed into the bone 64. Since more bone cement 74 than necessary is prevented from being injected into the bone 64, an accurate amount of bone cement 74 can be injected into the bone 64. After the inner needle 16 has been inserted again in the outer needle 12, the outer needle 12 and the inner needle 16 are pulled out of the bone 64 (see FIG. 6C).

With the puncture needle 10 according to the first unclaimed example, as described above, while the inner needle 16 is inserted in the outer needle 12, distal end portions of the outer needle 12 and the inner needle 16 are inserted into a target bone, after which the inner needle 16 is removed from the outer needle 12. Then, the inner tube 17 is inserted into the outer needle 12, whereupon the outer needle 12 and the inner tube 17 jointly make up double-tube constitution. The outer needle 12 includes the first side holes 22 and the second side holes 24 therein. When the inner tube 17 is inserted in the outer needle 12 and the outer needle 12 punctures the bone, the interior of the bone 64 and the space outside of the patient's body are held in fluid communication with each other through the first side holes 22, the depressurization passage 48, and the second side holes 24. Therefore, when bone cement is injected into the bone 64, since gas or liquid (e.g., exudate and blood) in the bone 64 can be made to flow out of the body through the depressurization passage 48, pressure buildup is prevented from developing in the bone 64 upon injection of bone cement into the bone 64, and as a result, bone cement is prevented from leaking out from the bone 64.

According to the first unclaimed example, since multiple first side holes 22 are provided, even if some of the first side holes 22 become clogged with liquid from within the bone, the liquid can flow through the other first side holes 22 and into the outer needle 12. Consequently, it is possible to prevent pressure buildup from developing in the bone more reliably.

The distance L3 is set to a value that is equal to or smaller than 20 mm, and more preferably, equal to or smaller than 15 mm, so that all of the first side holes 22 are positioned within the bone when the outer needle 12 punctures the bone. Accordingly, gas or liquid that has flowed from within the bone into the outer needle 12 is prevented from leaking out of the body through certain ones of the first side holes 22, which are positioned closer to the proximal end of the outer needle 12.

According to one proposal, the outer needle 12 may be of double-tube constitution, which is made up of an inner tube and an outer tube that are inseparable from each other, wherein the inner needle is insertable into the lumen of the inner tube. With such a proposal, however, it is difficult to increase the diameter of the inner needle due to the presence of the inner tube of the outer needle. According to the present invention, since the inner needle 16 is inserted into the outer needle 12 from which the inner tube 17 has been pulled out, the inner needle 16 can easily be increased in diameter for thereby enhancing the mechanical strength required for puncture and removal.

Since the flaring portion 28 is supported by the tapered support 30 in the outer needle hub 14, the outer needle 12 is prevented from being pulled out from the outer needle hub 14 upon removal of the puncture needle 10 from the bone 64.

Furthermore, since the outer needle hub 14 includes the auxiliary connection port 38, the puncture needle 10 can be cleaned easily and quickly by connecting a cleaning liquid injecting tool to the auxiliary connection port 38. A suction device can also be connected to the auxiliary connection port 38 to assist in discharging gas or liquid from the depressurization passage 48 of the puncture needle 10.

### [First Embodiment]

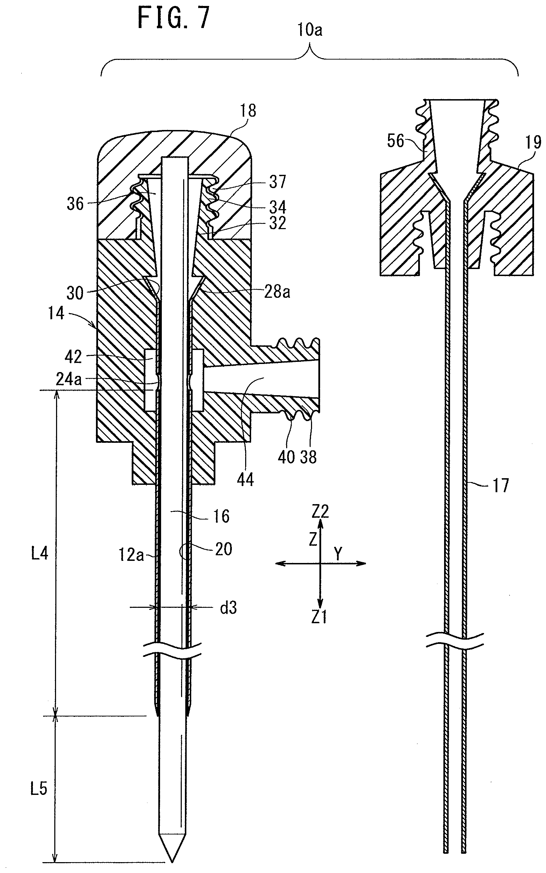

FIG. 7 is a cross-sectional view, partially omitted from illustration, of a bone cement injection puncture needle 10a (hereinafter referred to as a "puncture needle 10a") according to a first embodiment of the present invention. Parts of the puncture needle 10a according to the first embodiment, which function identically and have the same advantages as those of the puncture needle 10 according to the first unclaimed example, are donated by identical reference characters, and such features will not be described in detail below.

The puncture needle 10a according to the first embodiment includes an outer needle 12a, which replaces the outer needle 12 of the puncture needle 10 according to the first unclaimed example, and wherein the outer needle 12a is different in constitution from the outer needle 12. The outer needle 12a has a flaring portion 28a and side holes (proximal-end holes) 24a, which are constitutively identical to the flaring portion 28 and the second side holes 24 of the outer needle 12. However, the outer needle 12a is shorter than the outer needle 12, and does not include any constitution that corresponds to the first side holes 22 of the outer needle 12.

The distal end of the outer needle 12a is constructed as a sharp cutting edge, which enhances the ease with which bones can be punctured. The outer needle 12a may be made of any of the aforementioned materials, which have been described as making up the outer needle 12. The outer needle 12a has an outside diameter d3, which may be the same as the outside diameter d2 of the outer needle 12.

Regions of the second side holes 24a, which are located closest to the distal end of the outer needle 12a, are spaced from the most distal end portion of the outer needle 12a by a distance L4, which is set such that the side holes 24a remain reliably positioned outside of the patient's body when the outer needle 12a is inserted into a bone. More specifically, the distance L4 is equal to or greater than 100 mm, and more preferably, is equal to or greater than 110 mm. With the inner needle hub 18 connected to the outer needle hub 14, the length by which the inner needle 16 projects from the distal end of the outer needle 12a, i.e., the distance L5 between the distal end of the inner needle 16 and the distal end of the outer needle 12a, preferably is set to a value within a range from 2 to 15 mm.

Preferably, the outside diameter of the inner needle 16 is substantially the same as the inside diameter d3 of the outer needle 12a. More specifically, the outside diameter of the inner needle 16 may be set to a value that enables the inner needle 16 to be smoothly inserted into the lumen 20 of the outer needle 12a, with essentially no gap created between the outer circumferential surface of the inner needle 16 and the inner circumferential surface of the outer needle 12a.

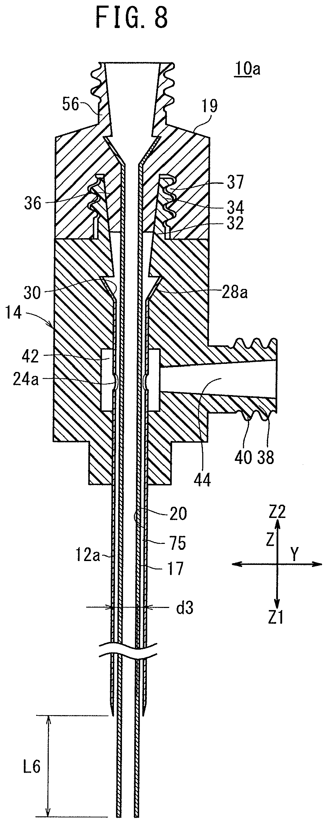

FIG. 8 is a cross-sectional view, partially omitted from illustration, of the bone cement injection puncture needle 10a, with the inner tube 17 being inserted in the outer needle 12a. As shown in FIG. 8, when the inner tube 17 is inserted into the outer needle 12a, a depressurization passage 75, which opens at the most distal end portion of the outer needle 12, is formed between the outer needle 12a and the inner tube 17. The depressurization passage 75 provides fluid communication between the distal end opening of the outer needle 12a and the side holes 24.

When the inner needle hub 18 is mounted on the outer needle hub 14, the inner tube 17 preferably should project from the distal end portion of the outer needle 12a. The length of the inner tube 17 that projects from the distal end of the outer needle 12a, i.e., the distance L6 between the distal end of the outer needle 12a and the distal end of the inner tube 17, preferably is set to a value within a range from 1 to 15 mm.

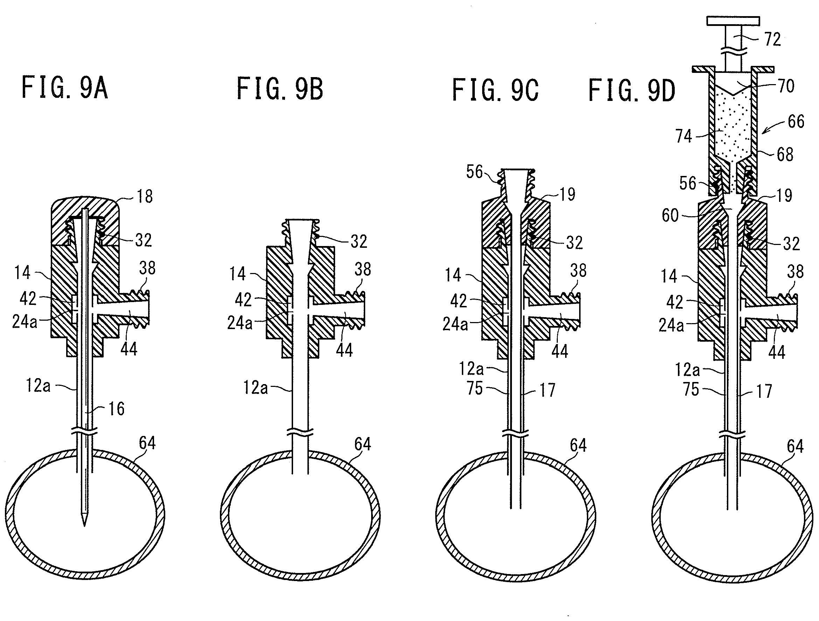

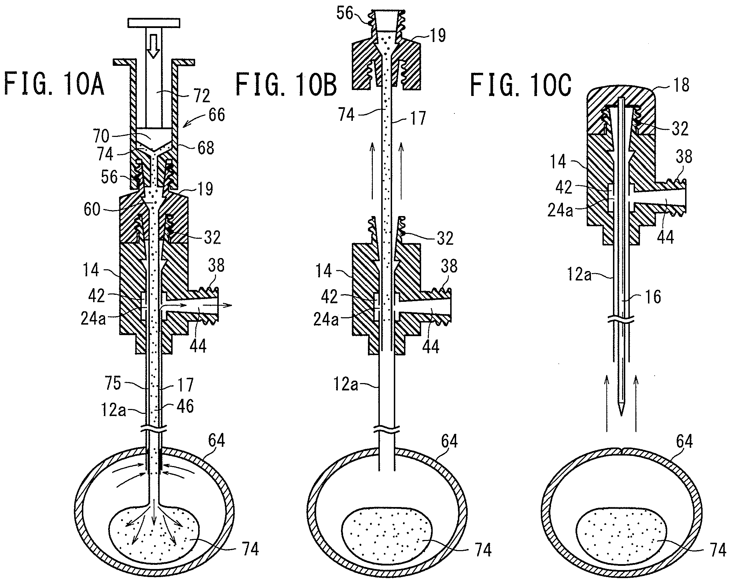

FIGS. 9A to 9D and FIGS. 10A to 10C are views illustrative of a process of injecting bone cement 74 into a bone 64 using the puncture needle 10a. For injecting bone cement 74 into the bone 64 using the puncture needle 10a, a puncture position and a puncture target are determined under image guidance. Thereafter, an assembly including the outer needle 12a and the outer needle hub 14, which are mounted respectively on the inner needle 16 and the inner needle hub 18, is hit by a hammer until the assembly is inserted into the bone 64, which serves as the puncture target (see FIG. 9A). At this time, the assembly is inserted in the bone 64 until the distal end opening of the outer needle 12a becomes positioned within the bone 64. The side holes 24a remain positioned outside of the bone when the outer needle 12a and the inner needle 16 are inserted in the bone 64.

As described above, when the inner tube 17 is inserted into the outer needle 12a, the distal end portion of the inner tube 17 projects from the distal end portion of the outer needle 12a. Therefore, when the outer needle 12a and the inner needle 16 are inserted into the bone under image guidance, the step between the distal end portion of the inner tube 17 and the distal end portion of the outer needle 12a serves as a marker. Since the step can easily be recognized visually in the image, the outer needle 12a can be inserted simply and reliably into the bone. If the step (marker) is confirmed based on X-rays, then the distal end of the depressurization passage 75 can also be judged as having punctured the bone.

After the outer needle 12a and the inner needle 16 have been inserted in the bone 64, the inner needle 16 is removed from the outer needle 12a while the outer needle 12a remains inserted in the bone 64 (see FIG. 9B). Then, the inner tube 17 is inserted into the outer needle 12a, and the inner tube hub 19 is connected to the main connection port 32 of the outer needle hub 14 (see FIG. 9C). The depressurization passage 75, which provides fluid communication between the interior of the bone 64 and the side holes 24 while the outer needle 12a is inserted in the bone 64, is formed between the outer needle 12a and the inner tube 17.

Then, a syringe 66, which comprises an injection device filled with bone cement 74, is connected to the injection port 56 (seed FIG. 9D). Then, the bone cement 74 in the syringe 66 is injected into the bone 64 through the lumen 60 of the inner tube hub 19 and the bone cement passage 46 (see FIG. 10A). At this time, gas or liquid in the bone 64, which corresponds in amount to the injected bone cement 74, flows from the distal end opening of the outer needle 12a into the depressurization passage 75, and then the gas or liquid flows into the depressurization passage 75 and out of the body through the side holes 24. Therefore, pressure buildup is prevented from developing in the bone 64 upon injection of bone cement 74 into the bone 64, and thus the bone cement 74 is prevented from leaking out from the bone 64.

A suction device, e.g., a syringe or the like, may be connected to the auxiliary connection port 38 in order to assist in discharging gas or liquid at the time that bone cement 74 is injected into the bone 64. Alternatively, while the inner tube 17 is inserted in the outer needle 12a, which has punctured the bone 64, a suction device may be connected to the auxiliary connection port 38 to allow gas or liquid in the bone 64 to be drawn out before bone cement 74 is injected into the bone 64, thereby developing a negative pressure in the bone 64, after which the bone cement 74 may be injected into the bone 64. In this manner, pressure in the bone 64 can be prevented from increasing upon injection of bone cement 74 into the bone 64.

After a predetermined amount of bone cement 74 has been injected into the bone 64, the inner tube 17 is pulled out from the outer needle 12a while the outer needle 12a remains inserted in the bone 64 (see FIG. 10B). At this time, bone cement 74 does not become adhered to the interior of the outer needle 12a, since the bone cement 74 is removed from within the outer needle 12a at the same time that the inner tube 17 is pulled out therefrom.

Then, the inner needle 16 is inserted again into the outer needle 12a, and the inner needle hub 18 is connected to the outer needle hub 14. At this time, no bone cement 74 remains within the outer needle 12a, as described above. Consequently, the inner needle 16 can reliably be inserted again into the outer needle 12a. When the inner needle 16 is inserted again, bone cement 74 is not pushed into the bone 64. Since more bone cement 74 than necessary is prevented from being injected into the bone 64, an accurate amount of bone cement 74 can be injected into the bone 64. After the inner needle 16 has been inserted again into the outer needle 12a, the outer needle 12a and the inner needle 16 are pulled out from the bone 64 (see FIG. 10C).

With the puncture needle 10a, as described above, while the inner needle 16 is inserted into the outer needle 12a, distal end portions of the outer needle 12a and the inner needle 16 are inserted into a target bone 64. Thereafter, the inner needle 16 is removed from the outer needle 12a, and then the inner tube 17 is inserted into the outer needle 12a, whereupon the outer needle 12a and the inner tube 17 jointly make up double-tube constitution. The outer needle 12a includes the side holes 24a therein. When the inner tube 17 is inserted in the outer needle 12a and the outer needle 12a punctures the bone 64, the interior of the bone 64 and a space outside of the patient's body are held in fluid communication with each other through the depressurization passage 75 and the side holes 24a. Therefore, when bone cement 74 is injected into the bone 64, since gas or liquid (e.g., exudate and blood) in the bone 64 can be made to flow out of the body through the depressurization passage 75, pressure buildup is prevented from developing in the bone 64 upon injection of bone cement 74 into the bone 64, and thus the bone cement 74 is prevented from leaking outside of the bone 64.

Components according to the first embodiment, which are the same as those according to the first unclaimed example, operate in an identical or similar manner, and offer identical or similar advantages to those of the components according to the first unclaimed example.

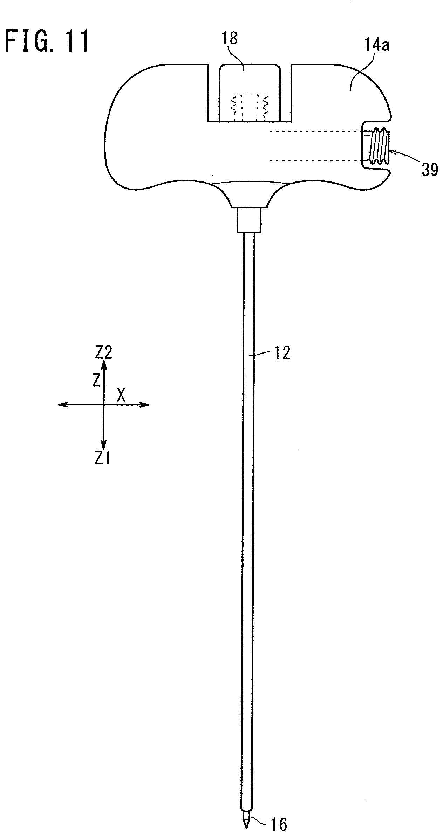

According to the first unclaimed example and the first embodiment, the outer needle hub 14 includes the auxiliary connection port 38 on one side surface thereof (a surface facing in a Y direction) . According to a second unclaimed example shown in FIG. 11, however, an outer needle hub 14a has an auxiliary connection port 39 provided on one of the ends thereof in the horizontal direction, i.e., on one of the ends thereof in the X directions. The auxiliary connection port 39 functions the same as the auxiliary connection port 38, and can be connected to another device or constitution, such as a suction device or the like.

According to percutaneous vertebroplasty, when a plurality of bone cement injection puncture needles are used, the puncture needles may be inserted into the body of a patient such that outer needle hubs thereof lie parallel to each other. The auxiliary connection port 39, which is disposed on a longitudinal end of the outer needle hub 14a as shown in FIG. 11, does not form an obstacle between adjacent puncture needles, thereby allowing the user to smoothly perform manual operations using the puncture needles.

### [Third unclaimed example]

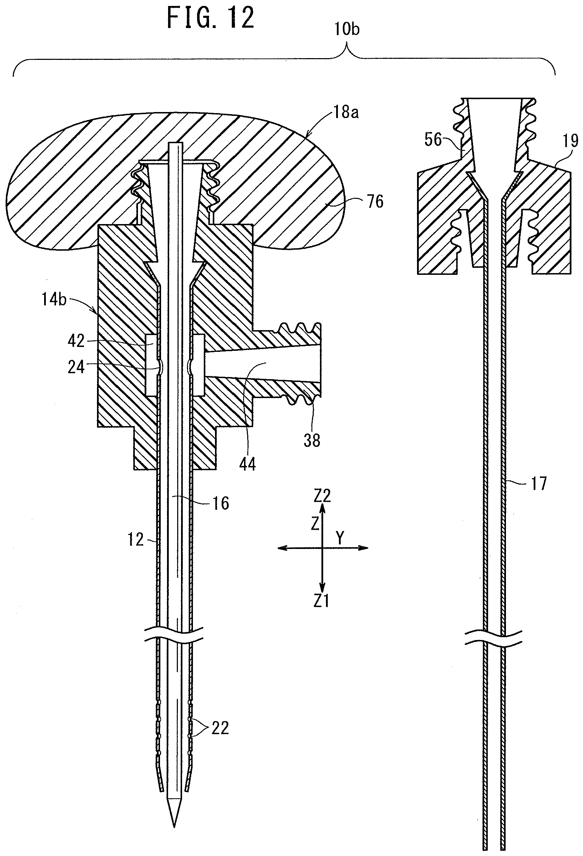

FIG. 12 is a cross-sectional view, partially omitted from illustration, of a bone cement injection puncture needle 10b (hereinafter referred to as a "puncture needle 10b") according to a third unclaimed example which does not form part of the invention but is useful for understanding the invention. Parts of the puncture needle 10b according to the third unclaimed example, which function identically and have the same advantages as those of the puncture needle 10 according to the first unclaimed example, are denoted by identical reference characters, and such features will not be described in detail below.

According to the first unclaimed example and the first embodiment, as described above, the grip 15, which is gripped by the user of the puncture needles 10, 10a, is included on the outer needle hub 14 (see FIG. 1). According to the third unclaimed example, however, a different grip 76, which extends in directions perpendicular to the axis of the inner needle 16, is included on an inner needle hub 18a, and the outer needle hub 14b does not include any constitution that corresponds to the grip 15 of the outer needle hub 14.

The outer needle hub 14b is similar in constitution to the outer needle hub 14, except that it is free of a grip. The outer needle hub 14b includes the first passage 36, the second passage 42, and the auxiliary connection port 38. The inner needle hub 18a is similar in constitution to the inner needle hub 18, except that it includes the grip 76. The outer needle 12 of the puncture needle 10b may also be replaced with the outer needle 12a of the puncture needle 10a according to the first embodiment.

With the puncture needle 10b according to the third unclaimed example, similar to the puncture needles 10, 10a according to the first unclaimed example and the first embodiment, after bone cement has been injected into the bone through the bone cement passage 46 in the inner tube 17, the inner tube 17 is pulled out from the outer needle 12. Therefore, bone cement does not become adhered to the interior of the outer needle 12. In view of this advantage, the grip 76 of the puncture needle 10b according to the third unclaimed example can be included on the inner needle hub 18a rather than the outer needle hub 14b. Specifically, since bone cement does not become adhered to the interior of the outer needle 12, the inner tube 17 can reliably be inserted again into the outer needle 12, and the grip 76 is included on the inner needle hub 18a to which the inner needle 16 is fixed. When bone cement is injected, the grip 76 is not removed from the outer needle hub 14. Consequently, even when the bone is punctured at a plurality of closely situated locations, the grip 76 does not present an obstacle when bone cement is injected, thereby allowing the user to smoothly perform manual operations on the puncture needles.

Components according to the third unclaimed example, which are the same as those according to the first unclaimed example, operate in an identical or similar manner, and offer identical or similar advantages to those of the components according to the first unclaimed example.

Although preferred embodiments of the present invention have been described above, it should be understood that the present invention is not limited to the above embodiments, but various changes and modifications may be made without departing from the scope of the present invention as set forth in the appended claims.

This application is a divisional application of European patent application no. 11 753 311.7 (the "parent application"), also published under no. EP 2 545 872. The following items corresponding to the originally filed claims of the parent application form part of the content of this description as filed.

### Items

Item 1. A bone cement injection puncture needle (10, 10a, 10b) comprising:
   an outer needle (12, 12a) of hollow constitution having a proximal end side hole (24, 24a) formed in a side surface near a proximal end portion thereof;
   an outer needle hub (14, 14a, 14b) fixed to the proximal end portion of the outer needle (12, 12a) and having a first port (32) held in fluid communication with a proximal end opening of the outer needle (12, 12a);
   an inner needle (16) having a needle point on a distal end thereof and which is insertable in the outer needle (12, 12a) and the first port (32);
   an inner needle hub (18, 18a) fixed to a proximal end portion of the inner needle (16) and which is removably mountable on the outer needle hub (14, 14a, 14b);
   an inner tube (17) insertable in the outer needle (12, 12a) and the first port (32); and
   an inner tube hub (19) fixed to a proximal end portion of the inner tube (17) and which is removably mountable on the outer needle hub (14, 14a, 14b) the inner tube hub (19) having a second port (56) held in fluid communication with a proximal end opening of the inner tube (17),
   wherein when the inner tube (17) is inserted in the outer needle (12, 12a) and the outer needle (12, 12a) punctures a bone, a flow passage, which provides fluid communication between the interior of the bone and the proximal end side hole (24, 24a), is formed between the outer needle (12, 12a) and the inner tube (17).
Item 2. The bone cement injection puncture needle (10, 10b) according to item 1, wherein the outer needle (12) has a distal end side hole (22) formed in a side surface near a distal end portion thereof; and
   when the inner tube (17) is inserted in the outer needle (12), a depressurization passage (48), which provides fluid communication between the distal end side hole (22) and the proximal end side hole (24), is formed between the outer needle (12) and the inner tube (17).
Item 3. The bone cement injection puncture needle (10, 10b) according to item 2, wherein when the inner tube hub (19) is mounted on the outer needle hub (14, 14a, 14b), a most distal end portion of the inner tube (17) is aligned with a most distal end portion of the outer needle (12) or projects from the outer needle (12, 12a).
Item 4. The bone cement injection puncture needle (10a) according to item 1, wherein when the inner tube (17) is inserted in the outer needle (12a), a depressurization passage (75), which opens at a most distal end portion of the outer needle (12a), is formed between the outer needle (12a) and the inner tube (17).
Item 5. The bone cement injection puncture needle (10a) according to item 4, wherein when the inner tube (17) is inserted in the outer needle (12a), a distal end portion of the inner tube (17) projects from a distal end portion of the outer needle (12a).

## Claims

1. A bone cement injection puncture needle (10a) comprising:
an outer needle (12a) of hollow constitution having a proximal end side hole (24a) formed in a side surface near a proximal end portion thereof;
an outer needle hub (14) fixed to the proximal end portion of the outer needle (12a) and having a first port (32) held in fluid communication with a proximal end opening of the outer needle (12a);
an inner needle (16) having a needle point on a distal end thereof and which is insertable in the outer needle (12a) and the first port (32); and
an inner needle hub (18) fixed to a proximal end portion of the inner needle (16) and which is removably mountable on the outer needle hub (14);
**characterized in that**
the bone cement injection puncture needle (10a) further comprises:
an inner tube (17) insertable in the outer needle (12a) and the first port (32); and
an inner tube hub (19) fixed to a proximal end portion of the inner tube (17) and which is removably mountable on the outer needle hub (14), the inner tube hub (19) having a second port (56) held in fluid communication with a proximal end opening of the inner tube (17),
wherein when the inner tube (17) is inserted in the outer needle (12a), a depressurization passage (75), which opens at a most distal end portion of the outer needle (12a) and provides fluid communication with the proximal end side hole (24a), is formed between the outer needle (12a) and the inner tube (17).

2. The bone cement injection puncture needle (10a) according to claim 1, wherein when the inner tube hub (19) is mounted on the outer needle hub (14), a most distal end portion of the inner tube (17) is aligned with a most distal end portion of the outer needle (12a).

3. The bone cement injection puncture needle (10a) according to claim 1, wherein when the inner tube (17) is completely inserted in the outer needle (12a), a distal end portion of the inner tube (17) projects from a distal end portion of the outer needle (12a).

4. The bone cement injection puncture needle (10a) according to any one of claims 1 to 3, wherein the first port (32) of the outer needle hub (14) has a first externally threaded surface (34),
the inner needle hub (18) has a first internally threaded surface (37), which is adapted to be screwed on the of the first externally threaded surface (34),
the inner needle hub (18) becomes fixed to the outer needle hub (14) when the inner needle hub (18) is screwed on the first port (32),
the inner tube hub (19) has a second internally threaded surface (54), which is adapted to be screwed on the of the first externally threaded surface (34), and
the inner tube hub (19) becomes fixed to the outer needle hub (14) when the inner tube hub (19) is screwed on the first port (32).

5. The bone cement injection puncture needle (10a) according to any one of claims 1 to 4, wherein the outer needle hub (14) includes a third port (38) that is held in fluid communication with a lumen (20) of the outer needle (12a) through the proximal end side hole (24a).

6. The bone cement injection puncture needle (10a) according to claim 5, wherein the third port (38) has a second externally threaded surface (40) at an outer circumference thereof for screwed-connection to another device or constitution.

## Patentansprüche

1. Knochenzementinjektionspunktiernadel (10a) mit:
einer Außennadel (12a) mit hohlem Aufbau mit einem an der proximalen Endseite befindlichen Loch (24a), das an einer Seitenfläche ausgebildet ist, die nahe ihrem proximalen Endabschnitt ist;
einem Außennadelansatz (14), der an dem proximalen Endabschnitt der Außennadel (12a) fixiert ist und eine erste Öffnung (32) hat, die in Fluidkommunikation mit einer proximalen Endöffnung der Außennadel (12a) gehalten ist;
einer Innennadel (16) mit einem Nadelpunkt an ihrem distalen Ende, die in die Außennadel (12a) und die erste Öffnung (32) einführbar ist; und
einem Innennadelansatz (18), der an einem proximalen Endabschnitt der Innennadel (16) fixiert ist und der an dem Außennadelansatz (14) entfernbar montierbar ist;
**dadurch gekennzeichnet, dass**
die Knochenzementinjektionspunktiernadel (10a) des Weiteren folgendes aufweist:
eine Innenröhre (17), die in die Außennadel (12a) und die erste Öffnung (32) einführbar ist; und
einen Innenröhrenansatz (19), der an einem proximalen Endabschnitt der Innenröhre (17) fixiert ist und der an dem Außennadelansatz (14) entfernbar montierbar ist, wobei der Innenröhrenansatz (19) eine zweite Öffnung (56) hat, die mit einer proximalen Endöffnung der Innenröhre (17) in Fluidkommunikation gehalten ist,
wobei, wenn die Innenröhre (17) in die Außennadel (12a) eingeführt ist, ein Druckentlastungskanal (75), der an einem am weitesten distal befindlichen Endabschnitt der Außennadel (12a) offen ist und eine Fluidkommunikation mit dem an der proximalen Endseite befindlichen Loch (24a) vorsieht, zwischen der Außennadel (12a) und der Innenröhre (17) ausgebildet ist.

2. Knochenzementinjektionspunktiernadel (10a) gemäß Anspruch 1, wobei, wenn der Innenröhrenansatz (19) an dem Außennadelansatz (14) montiert ist, ein am weitesten distal befindlicher Endabschnitt der Innenröhre (17) mit einem am weitesten distal befindlichen Endabschnitt der Außennadel (12a) ausgerichtet ist.

3. Knochenzementinjektionspunktiernadel (10a) gemäß Anspruch 1, wobei, wenn die Innenröhre (17) in der Außennadel (12a) vollständig eingeführt ist, ein distaler Endabschnitt der Innenröhre (17) von einem distalen Endabschnitt der Außennadel (12a) vorragt.

4. Knochenzementinjektionspunktiernadel (10a) gemäß einem der Ansprüche 1 bis 3, wobei die erste Öffnung (32) des Außennadelansatzes (14) eine erste mit einem Außengewinde versehene Fläche (34) hat,
der Innennadelansatz (18) eine erste mit einem Innengewinde versehene Fläche (37) hat, die daran angepasst ist, an der ersten mit dem Außengewinde versehenen Fläche (34) verschraubt zu werden,
der Innennadelansatz (18) an dem Außennadelansatz (14) fixiert wird, wenn der Innennadelansatz (18) auf die erste Öffnung (32) geschraubt ist,
der Innenröhrenansatz (19) eine zweite mit einem Innengewinde versehene Fläche (54) hat, die daran angepasst ist, an die erste mit dem Außengewinde versehene Fläche (34) geschraubt zu werden, und
der Innenröhrenansatz (19) an dem Außennadelansatz (14) fixiert wird, wenn der Innenröhrenansatz (19) auf die erste Öffnung (32) geschraubt wird.

5. Knochenzementinjektionspunktiernadel (10a) gemäß einem der Ansprüche 1 bis 4, wobei der Außennadelansatz (14) eine dritte Öffnung (38) hat, die mit einem Lumen (20) der Außennadel (12a) durch das an der proximalen Endseite befindliche Loch (24a) in Fluidkommunikation gehalten ist.

6. Knochenzementinjektionspunktiernadel (10a) gemäß Anspruch 5, wobei die dritte Öffnung (38) eine zweite mit einem Außengewinde versehene Fläche (40) an ihrem Außenumfang hat für eine Schraubverbindung mit einer anderen Vorrichtung oder einem anderen Aufbau.

## Revendications

1. Aiguille d'introduction pour injection de ciment osseux (10a) comprenant :
une aiguille externe (12a) de constitution creuse ayant un trou du côté de l'extrémité proximale (24a) formé dans une surface latérale à proximité de sa partie d'extrémité proximale ;
un raccord d'aiguille externe (14) fixé à la partie d'extrémité proximale de l'aiguille externe (12a) et ayant un premier orifice (32) maintenu en communication de fluide avec une ouverture d'extrémité proximale de l'aiguille externe (12a) ;
une aiguille interne (16) ayant une pointe d'aiguille sur son extrémité distale et qui peut être insérée dans l'aiguille externe (12a) et le premier orifice (32) ; et
un raccord d'aiguille interne (18) fixé à une partie d'extrémité proximale de l'aiguille interne (16) et qui peut être monté de manière amovible sur le raccord d'aiguille externe (14) ;
**caractérisée en ce que**
l'aiguille d'introduction pour injection de ciment osseux (10a) comprend en outre :
un tube interne (17) pouvant être inséré dans l'aiguille externe (12a) et le premier orifice (32) ; et
un raccord de tube interne (19) fixé à une partie d'extrémité proximale du tube interne (17) et qui peut être monté de manière amovible sur le raccord d'aiguille externe (14), le raccord de tube interne (19) ayant un deuxième orifice (56) maintenu en communication de fluide avec une ouverture d'extrémité proximale du tube interne (17),
dans laquelle, lorsque le tube interne (17) est inséré dans l'aiguille externe (12a), un passage de dépressurisation (75), qui s'ouvre au niveau de la partie d'extrémité la plus distale de l'aiguille externe (12a) et fournit la communication de fluide avec le trou du côté de l'extrémité proximale (24a), est formé entre l'aiguille externe (12a) et le tube interne (17).

2. Aiguille d'introduction pour injection de ciment osseux (10a) selon la revendication 1, dans laquelle, lorsque le raccord de tube interne (19) est monté sur le raccord d'aiguille externe (14), la partie d'extrémité la plus distale du tube interne (17) est alignée avec la partie d'extrémité la plus distale de l'aiguille externe (12a).

3. Aiguille d'introduction pour injection de ciment osseux (10a) selon la revendication 1, dans laquelle, lorsque le tube interne (17) est complètement inséré dans l'aiguille externe (12a), une partie d'extrémité distale du tube interne (17) fait saillie d'une partie d'extrémité distale de l'aiguille externe (12a).

4. Aiguille d'introduction pour injection de ciment osseux (10a) selon l'une quelconque des revendications 1 à 3, dans laquelle le premier orifice (32) du raccord d'aiguille externe (14) a une première surface extérieurement filetée (34),
le raccord d'aiguille interne (18) a une première surface intérieurement filetée (37) qui est adaptée pour être vissée sur la première surface extérieurement filetée (34),
le raccord d'aiguille interne (18) est fixé sur le raccord d'aiguille externe (14) lorsque le raccord d'aiguille interne (18) est vissé sur le premier orifice (32),
le raccord de tube interne (19) a une seconde surface intérieurement filetée (54) qui est adaptée pour être vissée sur la première surface extérieurement filetée (34), et
le raccord de tube interne (19) est fixé au raccord d'aiguille externe (14) lorsque le raccord de tube interne (19) est vissé sur le premier orifice (32).

5. Aiguille d'introduction pour injection de ciment osseux (10a) selon l'une quelconque des revendications 1 à 4, dans laquelle le raccord d'aiguille externe (14) comprend un troisième orifice (38) qui est maintenu en communication de fluide avec une lumière (20) de l'aiguille externe (12a) à travers le trou du côté de l'extrémité proximale (24a).

6. Aiguille d'introduction pour injection de ciment osseux (10a) selon la revendication 5, dans laquelle le troisième orifice (38) a une seconde surface extérieurement filetée (40) au niveau de sa circonférence externe pour le raccordement vissé à un autre dispositif ou une autre constitution.
